# EUROPÄISCHE PATENTSCHRIFT

(11) **EP 0 408 691 B1**
(45) Veröffentlichungstag und Bekanntmachung des Hinweises auf die Patenterteilung: **31.08.1994**
(21) Anmeldenummer: 90900071.3
(22) Anmeldetag: 21.12.1989
(51) Int. Cl.: A61K 37/18

(54) **NEPHROPROTEKTIVE INFUSIONSLÖSUNG**
ANTINEPHROTOXIC PERFUSION SOLUTION
SOLUTION DE PERFUSION ANTINEPHROTOXIQUE

(30) Priorität: 22.12.1988 DE 3843241
(43) Veröffentlichungstag der Anmeldung: 23.01.1991
(73) Patentinhaber: SKREZEK, Christian, D-24796 Gross-Nordsee (DE); BETERMANN, Hagen, D-24106 Kiel (DE)
(72) Erfinder: BERTERMANN, Hagen, D-2300 Kiel (DE)
(74) Vertreter: Tönnies, Jan G., Dipl.-Ing.
(86) Internationale Anmeldenummer: DE8900780
(87) Internationale Veröffentlichungsnummer: WO9006769

(56) Entgegenhaltungen:
- Patent Abstracts of Japan, vol. 12, no. 237 (C-509)(3084), 6 July 1988
- Patent Abstracts of Japan, vol. 12, no. 237 (C-509)(3084), 6 July 1988
- Chemical Abstracts, vol. 111, no. 18, 30 Oct. 1989, 8Columbus, Ohio, US), see p. 423, abstract no. 160229v

## Beschreibung

Die toxische Nierenschädigung stellt eine wesentliche Nebenwirkung der zytostatischen Therapie maligner Tumoren dar, die etwa ein Drittel der Patienten von der Therapie ausschließt. Infolge der Nephrotoxitität können weitere toxische Schäden an anderen Organen, insbesondere dem Knochenmark, auftreten, wenn die renale Elimination der Zytostatika oder Immunsuppressiva verzögert wird. Bestrebungen, durch Steigerung der Diurese und/oder des renalen Harnflusses die Toxizität zu vermindern, verhindern nicht, daß schwerwiegende Störungen der Nierenfunktion infolge der Gabe von Zystostatika oder Immunsupressiva auftreten. Dabei bleibt die renale Minderfunktion klinisch oft unentdeckt, weil die harnpflichtigen Substanzen im Blut erst bei höhergradigem Funktionsverlust der Nieren ansteigen.

Es ist ein bekanntes Verfahren, niereninsuffiziente Patienten unter nutritiven Gesichtspunkten mit Aminosäurengemischen, die vorwiegend essentielle L-Aminosäuren enthalten, zu behandeln.

Die in Patent der JP-A-6330411 und der JP-A-6330413 offenbarten Gemische von L-Aminosäuren wirken nachteilig auf die Niere. In Einzelfällen induzieren sie ein akutes Nierenversagen oder beeinflussen ein solches akutes Nierenversagen ungünstig. Zum Schutz der Niere gegen die toxische Wirkung von Zytostatika und Immunsuppressiva sind die bekannten Aminosäurengemische daher nicht geeignet.

Durch die Erfindung soll ein Gemisch aus neutralen Aminosäuren geschaffen werden, das bei Aufrechterhaltung einer hohen glomerulären Filtrationsrate im erforderlichen Dosisbereich des Gemisches eine Blockade der Lysosomenaktivität in der proximalen Tubuluszelle bewirkt.

Erfindungsgemäß wird diese Aufgabe gelöst durch ein Gemisch aus verschiedenen neutralen Aminosäuren zum Schutz der Nieren vor einer toxischen Schädigung durch nephrotoxische Zytostatika und Immunsuppressiva, das die folgende Zusammensetzung

| | |
|---|---|
| Glyzin: | 9 - 11 g/l |
| Alanin: | 12 - 17 g/l |
| Serin: | 10 - 18 g/l |
| Threonin: | 2 - 5 g/l |
| Valin: | 5 - 10 g/l |
| Leuzin: | 6 - 10 g/l |
| Isoleuzin: | 2 - 4 g/l |
| Prolin: | 6 - 12 g/l |

aufweist. Die Unteransprüche geben vorteilhafte Ausbildungen an.

Aufgrund der bewirkten Blockade der Lysosomenaktivität wird die proximale Tubuluszelle reversibel unempfindlich für eine Schädigung durch Zytostaika und Immunsuppressiva.

Es hat sich gezeigt, daß das hier vorgeschlagene Gemisch bei intravenöser Zuführung vor der zytostatischen Behandlung toxischen Nierenschädigungen durch die zytostatischen Medikamente oder aber bei der Gabe von Immunsuppressiva entgegenwirkt. Das hier vorgeschlagene Gemisch stabilisiert die renale Filtrationsfunktion und fördert die Energiegewinnung der Tubuluszelle während der Frühphase einer Nierenschädigung durch Zytostatika und Immunsuppressiva. Dieser zelluläre Angriffspunkt des Gemisches verhindert, daß die Zellfunktionsminderung zu einem irreversiblen Schaden der Nierenzelle mit nachfolgendem Zelluntergang führt.

Versuche haben gezeigt, daß die bloße Variation der vorbekannten Lösung nicht zum gewünschten Erfolg führt.

Die hier vorgeschlagene Verwendung zielt damit auf Nierengesunde ab, die bei der Behandlung einer anderen Krankheit dem Risiko einer Nierenschädigung unterliegen. Dagegen ist eine Ernährung mit Eiweißbausteinen mit dem hier vorgeschlagenen Gemisch nicht beabsichtigt, da diese anders sichergestellt wird. Die ausschließlich auf eine Toxizitätsblockade abzielende Zusammensetzung des Gemisches minimiert gleichzeitig die Stickstoff-Belastung. Der Protektionseffekt ist dabei von dem Konzentrationsverhältnis der verwendeten Aminosäuren untereinander abhängig.

Die Aufnahme der Aminosäuren in die Nierenzelle wurde anhand von Stoffwechseleffekten untersucht, sowohl bezüglich der einzelnen Aminosäuren als auch bezüglich verschiedener Kombinationen von Aminosäuren. Als Präparat dienten bei diesen Untersuchungen Suspensionen nativer Nieren-Tubulus-Segmente, die in oxygenierten Medien körperwarm inkubiert wurden.

Toxische Schädigungen der Niere durch Zytostatika wie Cisplatin und Carboplatin erfolgen im wesentlichen an der proximalen Tubuluszelle mit einem intrazellulären Angriffspunkt des nicht gebundenen Platins und seiner Metaboliten. Die Untersuchungen ergaben im Rahmen der Cisplatin-Nephrotoxizität eine Aktivierung des Autophagie-Systems. Weiter lassen die vorliegenden Ergebnisse darauf schließen, daß die Autophagie einer Regulation unterworfen ist, die durch L-Aminosäuren beeinflußt werden kann. Überraschenderweise konnte die Aktivierbarkeit des Systems durch nephrotoxische Zytostatika, insbesondere durch Cisplatin und Carboplatin, in Abhängigkeit von der gewählten L-Aminosäuren-Kombination sowohl in hemmender als auch in aktivierender Weise moduliert werden.Es zeigt sich dabei nur eine eingeschränkte Konzentrationsabhängigkeit, die durch intrazelluläre Kumulationseffekte von Aminosäuren erklärt werden kann. Diese entstehen, weil die Transportkinetik an der (urinseitigen) luminalen Membran schneller verläuft als an der (blutseitigen) basalen Membran. Parallel durchgeführte Experimente zeigten, daß die Stimulation der Autophagie mit einer Freisetzung lymosaler Enzyme verbunden ist, die ihrerseits eine Schädigung der Regulation der mitochondrialen Funktion bis zur vollständigen Entkopplung der sauerstoffabhängigen Energiegewinnung erzeugt (Beispiel 1). Eine hochgradige Schädigung der respiratorischen Mitochondrienfunktion führt zum Untergang proximaler Tubuluszellen.

Die hier vorgeschlagene Verwendung von neutralen L-Aminosäuren (Tab. 1) für nephroprotektive Zwecke beruht auf der Modulation der intrazellulären Autophagie in der Nierenzelle. Die Aktivierung des Autophagiesystems durch nephrotoxische Effekte ist reversibel blockiert, wenn das beschriebene L-Aminosäurengemisch zuvor appliziert wird.Die Zusammensetzung des Gemisches beruht auf systematischen Untersuchungen an proximalen Tubuluszellen der Niere. Der Vorteil der Verwendung eines Gemisches bestehend aus verschiedenen neutralen Aminosäuren liegt darin, daß in der Zelle ein Maximum an Wirkung dadurch erreicht wird, daß die einzelnen L-Aminosäuren des Gemisches über verschiedene Transportkanäle die Zellmembran passieren und sich intrazellulär sammeln. Als wesentliche Voraussetzung für eine erfolgreiche Toxizitätsblockade erwies sich eine intakte Nierenzellfunktion. Dies liegt vermutlich darin begründet, daß eine für die Schutzwirlung optimale intrazelläre L-Aminosäurenkonzentration von der Zelle selbst durch einen aktiven, Energie verbrauchenden Kumulationsvorgang erzeugt wird. Vor Einsetzen der nephrotoxischen Zytostatika-Effekte muß die Modulation der lysosomalen Autophagie erfolgt sein. Um die Modulation zu induzieren ist eine Zeitspanne von etwa 18 Stunden erforderlich. In vitro konnte sogar eine deutliche Förderung der Zellvitalität trotz gleichzeitiger Einwirkung von Zytostatika beobachtet werden.

**Tabelle 1**

| | g/l | mM/l |
|---|---|---|
| L-Glyzin | 9-11 | 120-146 |
| L-Alanin | 12-17 | 135-191 |
| L-Serin | 10-18 | 95-171 |
| L-Threonin | 2-5 | 17-42 |
| L-Valin | 5-10 | 43-85 |
| L-Leuzin | 6-10 | 46-76 |
| L-Isoleuzin | 2-4 | 15-30 |
| L-Prolin | 6-12 | 52-104 |

Unter in vivo Bedingungen erwies sich das in Tabelle 2 wiedergegebene Gemisch aus L-Aminosäuren bezüglich der renalen Filtration und Reabsorption als besonders geeignet.

**Tabelle 2**

| | g/l | mM/l |
|---|---|---|
| L-Glyzin | 11 | 146 |
| L-Alanin | 15 | 168 |
| L-Serin | 15 | 143 |
| L-Threonin | 5 | 42 |
| L-Valin | 10 | 85 |
| L-Leuzin | 10 | 76 |
| L-Isoleuzin | 4 | 30 |
| L-Prolin | 10 | 87 |

Im Unterschied zur Förderung der Zellvitalität durch Gemische von L-Aminosäuren nach Tab. 1, die über den Mechanismus einer Unterdrückung der Autophagie in der Nierenzelle erfolgt, liegt der Filtrationsförderung durch diese Gemische im Prinzip ein vaskulärer Angriffspunkt mit eingegrenztem Konzentrationsbereich zugrunde.

Anwendungen eines aufgrund dieser Ergebnisse zusammengestellten Gemisches von neutralen L-Aminosäuren zur Nephroprotektion unter Chemotherapie nach Tabelle 2 wurden an Patienten durchgeführt (siehe Beispiel 2, LSG III). Es wurden 200 g des Gemisches pro 24 Stunden kontinuierlich infundiert, so daß im gesamten Behandlungszeitraum eine annähernd konstante Konzentration der Aminosäuren im Serum erreicht wurde. Die Untersuchungen ergaben, daß bei so vorbehandelten Patienten eine wirksame Nephroprotektion gegen toxische Chemotherapieeffekte bestand. Eine Vergleichsgruppe erhielt während der Chemotherapie eine handelsübliche Aminosäurenlösung (siehe Beispiel 2, LSG I) in gleicher Dosis in der Zusammensetzung (in g/l) gemäß Tabelle 3.

Eine weitere Vergleichsgruppe wurde (unter sonst analogen Bedingungen) mit einer Modifikation des Gemisches gemäß Tab. 3 behandelt, welches die sauren und basischen Aminosäuren des Gemisches der LSG I nicht enthielt (s. Beispiel 2, LSG II).

**Tabelle 3**

| | g/l |
|---|---|
| L-Isoleuzin | 5,1 |
| L-Leuzin | 8,9 |
| L-Lysin | 7,0 |
| L-Methionin | 3,8 |
| L-Phenylalanin | 5,1 |
| L-Threonin | 4,1 |
| L-Tryptophan | 1,8 |
| L-Valin | 4,8 |
| L-Arginin | 9,2 |
| L-Glyzin | 7,9 |
| L-Alanin | 13,7 |
| L-Asparagin | 3,7 |
| L-Aspartinsäure | 1,3 |
| L-Cystein | 0,7 |
| L-Glutaminsäure | 4,6 |
| L-Ornithin | 3,2 |
| L-Prolin | 8,9 |
| L-Serin | 2,4 |
| L-Tyrosin | 1,3 |

Die Vergleichsgruppen (LSG I und LSG II) wiesen eine stabile Filtrationsleistung für maximal drei Tage auf, danach fiel die Filtrationsrate erheblich ab, die tubuläre Reabsorbtion ging zurück und die tubulären Enzymverluste vervielfachten sich. Die Elimination potentiell nephrotoxischer an- bzw. kationischer Aminosäuren aus dem Gemisch gemäß Tab. 3 zeigte in equivalenter Dosis keinen meßbaren Protektionseffekt auf die Nierenfunktion unter Chemotherapie im Vergleich zum handelsüblichen Gemisch gemäß Tab. 3 (s. Beispiel 2, LSG II).

Demgegenüber zeichnete sich das Gemisch nach Tabelle 2 (LSG III) in der Anwendung bei Patienten durch eine gesteigerte renale Filtration während der gesamten Chemotherapie, eine normale Reabsorption und eine fast vollständige Normalisierung tubulärer Enzymlecks aus. Patienten, die ohne Nephroprotektion von der Chemotherapie ausgeschlossen worden wären, weil mit einem Nierenversagen durch die Chemotherapie gerechnet werden müßte, wiesen, soweit meßbar, keine nephrotoxischen Effekte auf. Darüber hinaus wurde trotz Chemotherapie eine wesentliche Verbesserung der Ausscheidungsfunktion der Niere gegenüber der Ausgangsleistung und ein Rückgang der harnpflichtigen Substanzen unter Anwendung des L-Aminosäurengemisches nach Tabelle 2 (LSG III) beobachtet. Zum Nachweis der Wirkung der beanspruchten Gemische auf die Nierenzellfunktion wurde neben den in der Klinik bekannten Methoden auch eine neu entwickelte Harnenymanalyse herangezogen, die eine wesentlich größere Empfindlichkeit für den Nachweis renaler Zellfunktionsstörungen besitzt. Mit hoch empfindlichen Harnenzymanalysen war die Blockade einer toxischen Schädigung de Nierenzellen in der mittels L-Aminosäurengemische geschützten Gruppe deutlich nachweisbar (siehe Beispiel 2).

Da das Gemisch nach Tabelle 2 ausschließlich aus neutralen Aminosäuren kombiniert ist, entstehen praktisch keine lösungstechnischen Imbalancen. Aus praktischen klinischen Gründen werden die Aminosäuren in 0,45 %iger Kochsalzlösung in Lösung gebracht, wobei Chlorid teilweise unter pH-Äquilibrierung durch Aspartat ersetzt wird.

**Tabelle 4**

| | g/l | mM/l |
|---|---|---|
| L-Glyzin | 11 | 146 |
| L-Alanin | 15 | 168 |
| L-Serin | 15 | 143 |
| L-Threonin | 5 | 42 |
| L-Valin | 10 | 85 |
| L-Leuzin | 10 | 76 |
| L-Isoleuzin | 4 | 30 |
| L-Prolin | 10 | 87 |
| Natriumchlorid/Aspartat | 4,5 | 80 |
| Summe | 85 | 857 |

Die Gemische der Tabellen 2 und 4 zeigten eine nephroprotektive Wirksamkeit gegen nephrotoxische zytostatische (Beispiel 2) und immunsuppressive Pharmaka (Beispiel 3). Die Beobachtungen ergaben, daß auch die Nebenwirkungen der selbst nicht nephrotoxischen, aber im wesentlichen über die Niere ausgeschiedenen Chemotherapeutika auf andere Organe, z.B. das blutbildende System, durch die genannten L-Aminosäuregemische unterdrückt wurden.

### Beispiel 1

### Freisetzung von Autophagieenzymaktivität und Regulationsstörung der respiratorischen Mitochondrienfunktion

Isolierte proximale Tubulussegmente (ITS) der Rattenniere wurden unterschiedlichen Cisplatin-Konzentrationen ausgesetzt, anschließend gewaschen und cisplatinfrei reinkubiert (je 20 min). Die ITS wurden in einem albuminhaltigen (10 %) Ringermedium suspendiert, dem als Substrate Glukose (Gluc) und Aminosäuren (AS) zugesetzt waren. Die in der Reinkubationsperiode beobachteten Leckraten der N-Acetyl-β-D-Glucosaminidase (NAG) sind dem mitochondrialen Akzeptorkontrollindex (ACI) zugeordnet. Als Kontrollen dienten während des Tests cisplatin-frei inkubierte ITS.
Gluc: 18 mM/l, Gluc + AS: 10 mM/l Glukose + insgesamt 8 mM/l GLY, ALA, SER, THR, VAL, LEU, ILE, PRO.
n = 12, x ± SEM,

| TEST | MEDIUM | NAG [U/g Protein/min] | ACI [± ADP] |
|---|---|---|---|
| | Gluc + AS | 4,3 ± 0,7 | 7,2 ± 1,4 |
| Kontrolle | Gluc | 4,7 ± 0,7 | 6,8 ± 1,2 |
| Cisplatin (5 mg %) | Gluc + AS | 10,3 ± 1,8 | 5,8 ± 1,0 |
| | Gluc | 17,5 ± 2,9 | 5,1 ± 0,8 |
| Cisplatin (10 mg %) | Gluc + AS | 31,3 ± 5,4 | 1,9 ± 0,3 |
| | Gluc | 35,2 ± 5,6 | 1,4 ± 0,3 |

### Beispiel 2

### Blockade nephrotoxischer Effekte von Platinderivaten mittels nephroprotektiver L-Aminosäuren

Bei 60 Patienten wurden vor (Interne Kontrolle) und während der zytostatischen Therapie mit Cis- und Carboplatin Parameter der tubulären (N-Acetyl-β-D-glucosaminidase, NAG, kumulativ) und der glomerulären (glomeruläre Filtrationsrate, GFR) Nierenfunktion gemessen. Je 12 Patienten erhielten eine Vorbehandlung mit einem nephroprotektiven Aminosäurengemisch nach Tabelle 2 (LSG III). Je 12 Patienten wurden mit einem handelsüblichen, konventionellen Aminosäurengemisch gemäß Tabelle 3 behandelt (LSG I). 12 weitere Patienten erhielten eine Modifikation des Gemisches gemäß Tabelle 3 ohne die Mischungskomponenten Glutaminsäure, Asparginsäure, Asparagin, Arginin, Lysin, Ornithin (LSG II).
x ± SEM.

| Zytostatikum | Aminosäurengemisch | cum NAG i.U. [Units] | GFR [ml/min] |
|---|---|---|---|
| Cisplatin | Interne Kontr. | 17,2 ± 2,4 | 115 ± 14 |
| | LSG I | 56,1 ± 7,1 | 55 ± 12 |
| | LSG II | 58,5 ± 6,3 | 59 ± 12 |
| | LSG III | 29,5 ± 4,9 | 180 ± 21 |
| Carboplatin | Interne Kontr. | 17,5 ± 2,3 | 112 ± 12 |
| | LSG I | 38,3 ± 5,4 | 68 ± 11 |
| | LSG III | 21,3 ± 3,9 | 186 ± 19 |

### Beispiel 3

### Blockade nephrotoxischer Effekte von Cyclosporin A mittels nephroprotektiver L-Aminosäuren

Bei 30 organtransplantierten Patienten wurden vor (Interne Kontrolle) und während der immunsuppressiven Therapie mit Cyclosporin A Parameter der tubulären (N-Acetyl-β-D-Glucosaminidase, NAG, berechnet auf Gramm Kreatinin im Urin) und der glomerulären (glomeruläre Filtrationsrate, GFR) Nierenfunktion gemessen. 10 Patienten erhielten eine Vorbehandlung mit einem nephroprotektiven Aminosäurengemisch gemäß Tabelle 2 (LSG III). 10 Patienten wurden mit einem handelsüblichen, konventionellen Aminosäurengemisch nach Tabelle 3 behandelt (LSG I). 10 Patienten erhielten eine Modifikation des Gemisches gemäß Tabelle 3 ohne die Mischungskomponenten L-Glutaminsäure, L-Asparaginsäure, L-Aspargin, L-Arginin, L-Lysin und L-Ornithin (LSG II).
x ± SEM.

| Immunsuppressivum | Aminosäurengemisch | NAG i.U. [U/g Kreatinin] | GFR [ml/min] |
|---|---|---|---|
| Cyclosporin A | Int. Kontr. | 2,8 ± 1,2 | 115 ± 14 |
| | LSG I | 23,1 ± 3,4 | 76 ± 12 |
| | LSG II | 24,5 ± 3,7 | 74 ± 12 |
| | LSG III | 12,5 ± 2,6 | 172 ± 22 |

## Patentansprüche

1. Gemisch aus verschiedenen neutralen Aminosäuren zum Schutz der Nieren vor einer toxischen Schädigung durch nephrotoxische Zytostatika und Immunsuppressiva, gekennzeichnet durch die folgende Zusammensetzung:
| | |
|---|---|
| Glyzin: | 9 - 11 g/l |
| Alanin: | 12 - 17 g/l |
| Serin: | 10 - 18 g/l |
| Threonin: | 2 - 5 g/l |
| Valin: | 5 - 10 g/l |
| Leuzin: | 6 - 10 g/l |
| Isoleuzin: | 2 - 4 g/l |
| Prolin: | 6 - 12 g/l |

2. Gemisch nach Anspruch 1, gekennzeichnet durch eine intravenös zuzuführende Formulierung in etwa 8%iger wäßriger Lösung.

3. Gemisch nach einem der vorangehenden Ansprüche, dadurch gekennzeichnet, daß die Aminosäuren in 0,45%iger Kochsalzlösung in Lösung gebracht sind.

4. Gemisch nach Anspruch 3, dadurch gekennzeichnet, daß das Chlorid der Kochsalzlösung teilweise unter pH-Äquilibrierung durch Aspartat ersetzt ist.

## Claims

1. Mixture of different neutral amino acids for nephroprotection from damage by nephrotoxic cytostatic and immunosuppressive agents, characterized by the following components:
| | |
|---|---|
| glycine: | 9-11 g/l |
| alanine: | 12-17 g/l |
| serine: | 10-18 g/l |
| threonine: | 2- 5 g/l |
| valine: | 5-10 g/l |
| leucine: | 6-10 g/l |
| isoleucine: | 2- 4 g/l |
| proline: | 6-12 g/l |

2. Mixture according to claim 1, characterized by an intravenously accessed formulation in approximately 8 % aqueous solution.

3. Mixture according to one of the preceding claims, characterized in that the amino acids be dissolved into a 0.45 % sodium chloride solution.

4. Mixture according to claim 3, characterized in that the chloride of the sodium chloride solution is partly replaced by aspartate while controlling pH values.

## Revendications

1. Mélange de divers aminoacides neutres, pour protéger les reins contre les effets toxiques des cytostatiques et immunosuppresseurs néphrotoxiques, caractérisé par la composition suivante :
| | |
|---|---|
| glycine | 9 à 11 g/l |
| alanine | 12 à 17 g/l |
| sérine | 10 à 18 g/l |
| thréonine | 2 à 5 g/l |
| valine | 5 à 10 g/l |
| leucine | 6 à 10 g/l |
| isoleucine | 2 à 4 g/l |
| proline | 6 à 12 g/l. |

2. Mélange selon la revendication 1, caractérisé par une formulation pour l'administration intraveineuse en solution aqueuse à une concentration d'environ 8%.

3. Mélange selon une des revendications qui précèdent, caractérisé en ce que les aminoacides sont dissous dans une solution de chlorure de sodium à 0,45 %.

4. Mélange selon la revendication 3, caractérisé en ce que le chlore de la solution de chlorure de sodium est remplacé en partie, avec équilibrage du pH, par un aspartate.
